# EUROPÄISCHE PATENTSCHRIFT

(11) **EP 1 035 873 B1**
(45) Veröffentlichungstag und Bekanntmachung des Hinweises auf die Patenterteilung: **05.06.2002**
(21) Anmeldenummer: 98965723.4
(22) Anmeldetag: 30.11.1998
(51) Int. Cl.: A61L 2/00

(54) **STERILISIERBEHÄLTER**
STERILIZING CONTAINER
RECIPIENT STERILISATEUR

(30) Priorität: 03.12.1997 DE 19753671
(43) Veröffentlichungstag der Anmeldung: 20.09.2000
(73) Patentinhaber: Wagner GmbH Fabrik für medizinische Geräte, 80634 München (DE)
(72) Erfinder: WAGNER, Peter, D-82319 Starnberg (DE)
(74) Vertreter: Koch, Günther, Dipl.-Ing.
(86) Internationale Anmeldenummer: EP9807746
(87) Internationale Veröffentlichungsnummer: WO9927969

(56) Entgegenhaltungen:
- EP-A- 0 152 544
- DE-A- 3 438 463
- DE-B- 1 217 551
- DE-C- 4 125 673
- US-A- 5 019 345

## Beschreibung

Die Erfindung betrifft einen Sterilisierbehälter zur Aufnahme von OP-Instrumenten oder anderweitigem Sterilisiergut während der Sterilisation und zur Sterilitätserhaltung während der Lagerung und beim Transport. Derartige Sterilisierbehälter müssen während der Sterilisation einen Medienaustausch innerhalb des Sterilisators ermöglichen.

Es ist bekannt, zur Ermöglichung des Medienaustausches die Sterilisierbehälter mit Doppelventilen auszustatten, die während des Sterilisiervorganges innerhalb des Sterilisators abwechselnd, je nach der auftretenden Druckdifferenz, öffnen und schließen und nach dem Sterilisiervorgang geschlossen bleiben, um das Sterilgut während der Lagerung gegen das Eindringen von Mikroorganismen zu schützen. Derartige Sterilisierbehälter sind beispielsweise aus der DE-AS 12 17 550, 12 17 551 sowie den DE-PS 16 42 161 und 32 02 430 bekannt. Andere bekannte Sterilisierbehälter weisen Behälteröffnungen auf, die mit einem Filter ausgestattet sind, durch den während des Sterilisiervorganges im Sterilisator der Medienaustausch erfolgt, und die nach Abschluß des Sterilisiervorganges während der Lagerung des Sterilisierbehälters eine Barriere gegen das Eindringen von Mikroorganismen bilden sollen. Derartige Sterilisierbehälter sind beispielsweise aus der DE 34 38 463 C2 bekannt. Aus dieser Patentschrift ist es auch bekannt, ein Filterblatt und ein unter Federvorspannung stehendes Filterhalteblech derart zu lagern, daß beim Überschreiten eines vorbestimmten Druckunterschiedes das Filterblatt von den Deckelöffnungen unter Freigabe eines Nebenstrompfades ventilartig abhebbar ist. Es wird dabei ein Bypass freigegeben, sobald der Außendruck so hoch wird, daß die Gefahr eines Zusammenquetschens des Behälters besteht.

Ein derartiges Überdruckventil sieht auch die DE 41 25 673 C1 vor. Dieses Überdruckventil, welches beim Auftreten eines vorbestimmten äußeren Druckes nach dem Behälterinneren öffnet, ist in eine Führung an der Behälterwand einschiebbar. In eine ähnliche Führung ist ein Filter einschiebbar, der einer weiteren Behälteröffnung zugeordnet ist.

Diese aus der DE 41 25 673 C1 bekannte und andere Ventil-Filter-Kombinationen sind allein als Schutzfunktion vorgesehen, um zu verhindern, daß der Behälter zusammengequetscht wird.

Bestimmungsgemäß erfolgte bei mit Filtern ausgestatteten Sterilisierbehältern der Medienaustausch im Sterilisator durch diese Filter hindurch. Die Filter waren demgemäß so ausgelegt, daß sie die auftretenden Druckunterschiede genügend schnell ausgleichen konnten. Dies bedingte eine entsprechende Porengröße, die zwar klein genug war, um den Behälterinhalt vor Staub und großvolumigen Keimen zu schützen, jedoch nicht sicherstellen konnten, daß keine Mikroorganismen - speziell auch extrem kleine Viren oder Pilzsporen - eindringen. Bei den mit Filtern ausgerüsteten Sterilisierbehältern bestand daher im Laufe der Zeit ein sich erhöhendes Risiko der Penetration von Mikroorganismen.

Der Erfindung liegt daher die Aufgabe zugrunde, einen mit Filter ausgerüsteten Sterilisierbehälter zu schaffen, bei dem der Filter feinporig und derart ausgebildet ist, daß das Eindringen von Mikroorganismen nach der Sterilisation auch bei längerer Lagerung nicht zu befürchten ist, wobei jedoch die bei modernen Autoklaven auftretenden, kurzzeitig wirksam werdenden Druckdifferenzen ohne Schaden für Filter oder Behälter abgebaut werden können.

Gelöst wird die gestellte Aufgabe durch die Gesamtheit der im Patentanspruch 1 angegebenen Merkmale.

Erfindungsgemäß werden somit zusätzlich zu dem Filter Ein- und Auslaßventile vorgesehen, die bestimmungsgemäß auch während des Sterilisiervorganges wirksam sind und nicht nur als Sicherheitsventile dienen, und die wirksam werden, sobald der Druckunterschied von außen nach innen oder von innen nach außen so groß geworden ist, daß der Medienaustausch durch das Filtermaterial selbst nicht schnell genug erfolgen kann.

Der Erfindung liegt demgemäß die Erkenntnis zugrunde, daß Sterilisierbehälter mit beliebig feinen Filtern ausgerüstet werden können, wenn dafür Sorge getragen wird, daß ein Bypass in beiden Richtungen, d.h. nicht nur von außen nach innen, sondern auch von innen nach außen geschaffen wird, wenn eine durch die Konstruktion vorbestimmte Druckdifferenz überschritten wird.

Gemäß einer bevorzugten Ausführungsform der Erfindung sind Filter und Ventile in einer gemeinsamen Behälteröffnung, beispielsweise in einer Öffnung eines mit Schutzdeckel versehenen Zwischendeckels oder in einer Behälterwand oder im Boden angeordnet. Dabei ergibt sich ein besonders zweckmäßiger und robuster Aufbau, wenn der Filter in einem Halterahmen unverrückbar eingespannt wird und wenigstens eines der Ventile im Halterahmen integriert ist, der seinerseits fest mit dem Öffnungsrand verspannt ist. Es soll für die Erfindung jedoch auch vorbehalten bleiben, Filter und wenigstens eines der Ventile in verschiedenen Behälteröffnungen anzuordnen, was beispielsweise dann zweckmäßig sein kann, wenn am Boden des Sterilisierbehälters ein Kondensat-Ablaßventil angeordnet werden soll. Dieses Kondensat-Ablaßventil kann dann gleichzeitig als Überdruckventil ausgebildet sein, über das ein erhöhter Behälterinnendruck abgebaut werden kann.

Die Integration der beiden Ventile im Halterahmen des Filters kann in der Weise erfolgen, daß keine nach außen hin beweglichen Teile vorhanden sind, die mit dem Behälterinhalt kollidieren könnten oder die von außen her manipulierbar sind.

Gemäß der Erfindung kann das Filtermaterial einen nahezu beliebigen Strömungswiderstand aufweisen, und es kann nichts verrutschen, wodurch die Gefahr ausgeschaltet wird, daß nach Vollendung des Sterilisationsvorganges ein offener Bypass verbleibt.

Gegenüber den bekannten abgefederten Filterhalteblechen, bei denen der Filter während des Auftretens der Bypass-Strömung geöffnet ist und durch die Strömung in eine andere Lage verrutschen kann oder sich verwellt, dichtet bei der erfindungsgemäßen Anordnung der Filter immer korrekt ab. Nach der Erfindung ist die gesamte Anordnung (Bypass mit Filter) unbeweglich eingebaut, was einen zweifachen Sicherheitsvorteil ergibt:
a) der Bypass kann nicht durch den Behälterinhalt in seiner Funktion blockiert werden;
b) es kann weder durch den Inhalt des Behälters, noch von außen her der Bypass unbeabsichtigt betätigt werden.

Gemäß der Erfindung können alle bekannten Filtermaterialien Verwendung finden, beispielsweise Flächenfilter aus Papier, Kunststoff oder Textilgewebe, aber auch Membranfilter oder 3D-Volumenfilter sowie Schwebstoffilter, durch die selbst Viren oder Pilzsporen abgehalten werden können.

Zweckmäßige Ausbildungen der Filter-Ventil-Kombination ergeben sich aus den Unteransprüchen und der Beschreibung von Ausführungsbeispielen.

Nachstehend werden Ausführungsbeispiele der Erfindung anhand der Zeichnung beschrieben. In der Zeichnung zeigen:
Fig. 1 zeigt einen Halbschnitt eines erfindungsgemäßen Sterilisierbehälters mit einem in einem Zwischendeckel eingebauten Filterblatt mit Doppelventilanordnung;
Fig. 2 ist eine Schnittansicht des Zwischendeckels gemäß Fig. 1;
Fig. 3 ist eine Ansicht der Filter-Ventilanordnung gemäß Fig. 2 von oben her betrachtet;
Fig. 4 ist eine Ansicht der Filter-Ventilanordnung gemäß Fig. 2 von unten her betrachtet;
Fig. 5 zeigt eine perspektivische Darstellung des den Filter umschließenden Ventilrings;
   die Fig. 6 bis 9 zeigen eine den Fig. 1 bis 5 ähnliche Filter-Ventil-Kombination, bei der jedoch das obere Filterhalteblech durch den Zwischendeckel selbst gebildet ist;

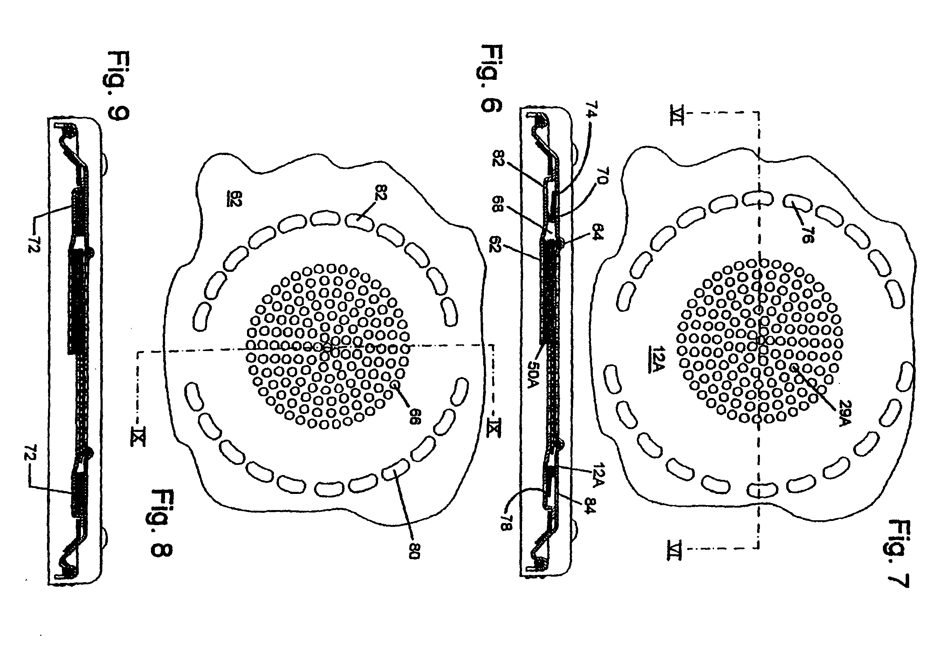

Fig. 6 zeigt einen Schnitt längs der Linie VI-VI gemäß Fig. 7;
Fig. 7 ist eine Draufsicht zu Fig. 6;
Fig. 8 ist eine Ansicht der Fig. 6 von unten her, und
Fig. 9 ist ein Schnitt längs der Linie IX-IX gemäß Fig. 8;

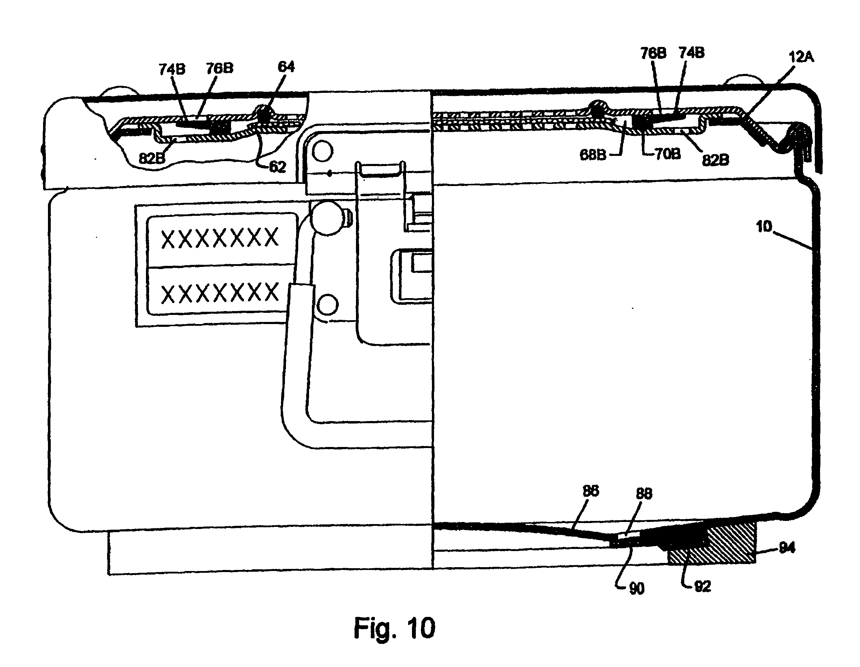

Fig. 10 ist eine Teilschnittansicht eines Sterilisierbehälters, der im Zwischendeckel mit einer Filter-Einlaßventil-Kombination versehen ist und das Auslaßventil im Bodenteil trägt;

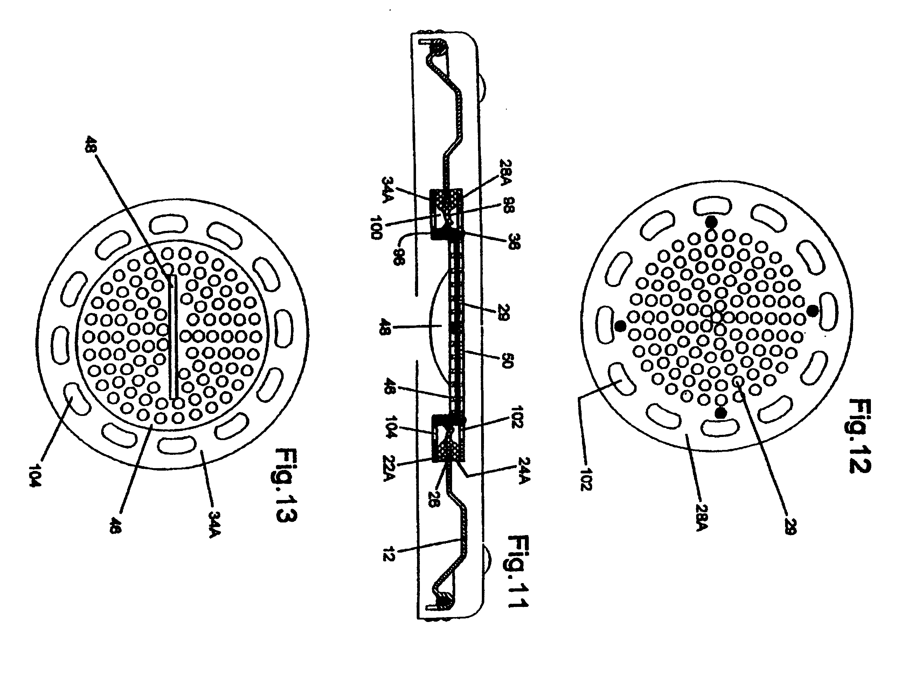

Fig. 11 zeigt eine Querschnittsansicht einer abgewandelten Ausführungsform einer im Zwischendeckel angeordneten Filter-Ventil-Kombination;
Fig. 12 ist eine Draufsicht der Filter-Ventil-Kombination von oben, und
Fig. 13 ist eine Draufsicht auf die Filter-Ventil-Kombination gemäß Fig. 11 von unten her betrachtet;

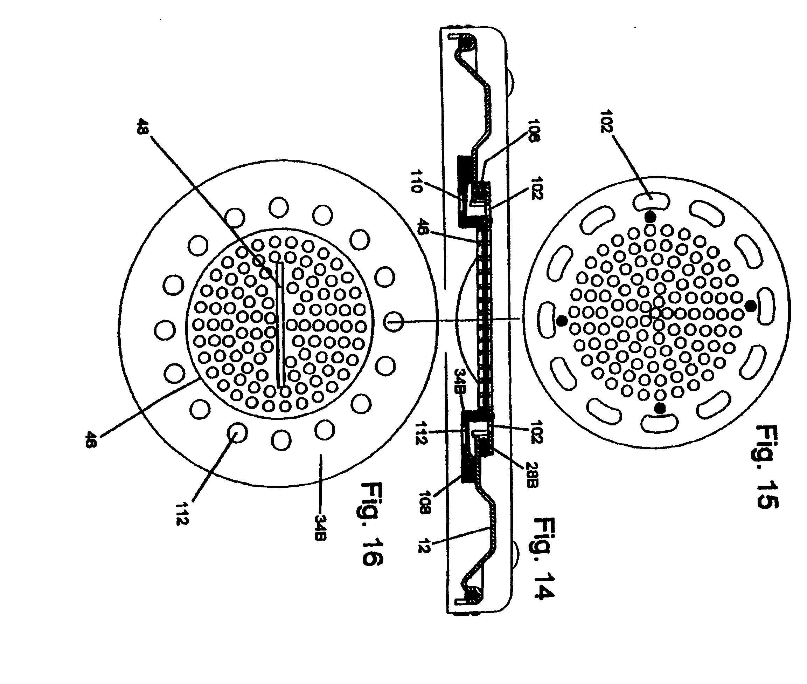

Fig. 14 ist eine Querschnittsansicht einer weiteren abgewandelten Ausführungsform einer Filter-Ventil-Kombination;
Fig. 15 ist eine Draufsicht gemäß Fig. 14 von oben her betrachtet;
Fig. 16 ist eine Draufsicht gemäß Fig. 14 von unten her betrachtet;

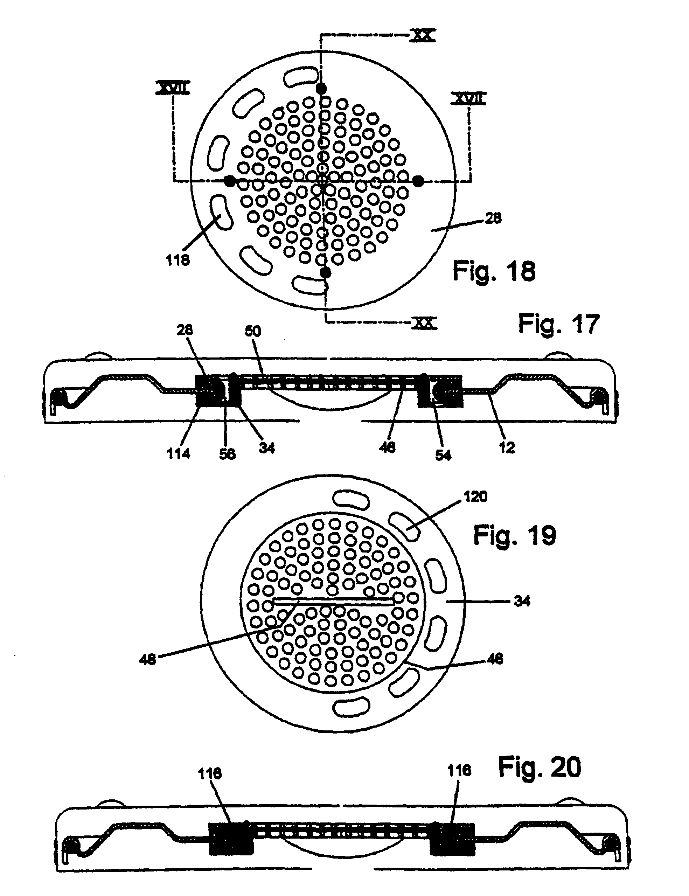

Fig. 17 ist eine Schnittansicht einer weiteren Ausführungsform einer Filter-Ventil-Kombination, geschnitten nach der Linie XVII-XVII gemäß Fig. 18;
Fig. 18 ist eine Ansicht der Filter-Ventil-Kombination gemäß Fig. 17 von oben her betrachtet;
Fig. 19 ist eine Ansicht der Filter-Ventil-Kombination gemäß Fig. 17 von unten her betrachtet;
Fig. 20 ist ein Schnitt nach der Linie XX-XX gemäß Fig. 18.

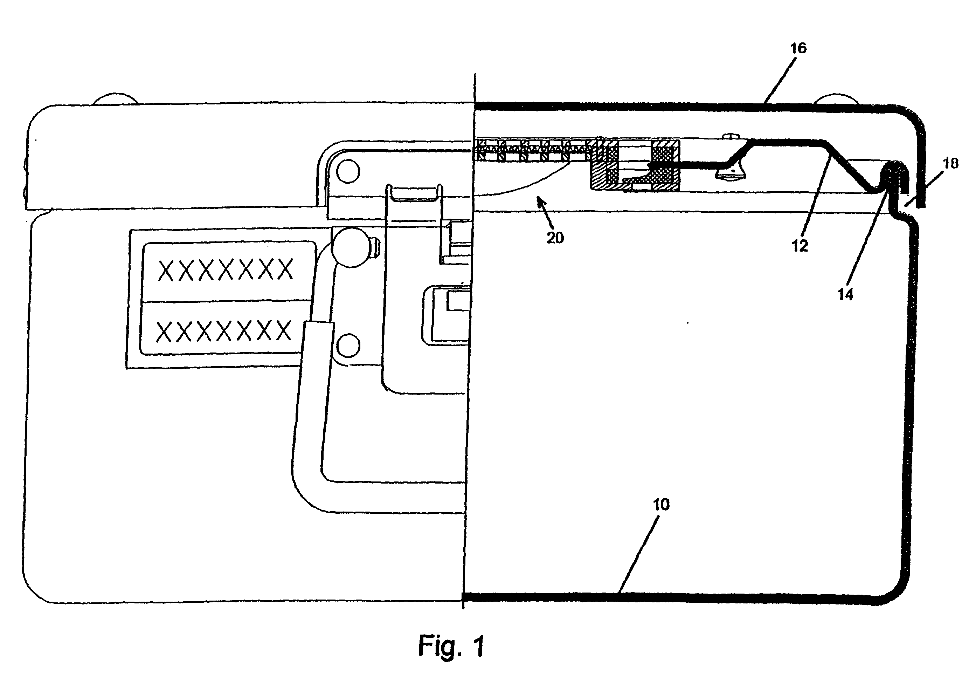

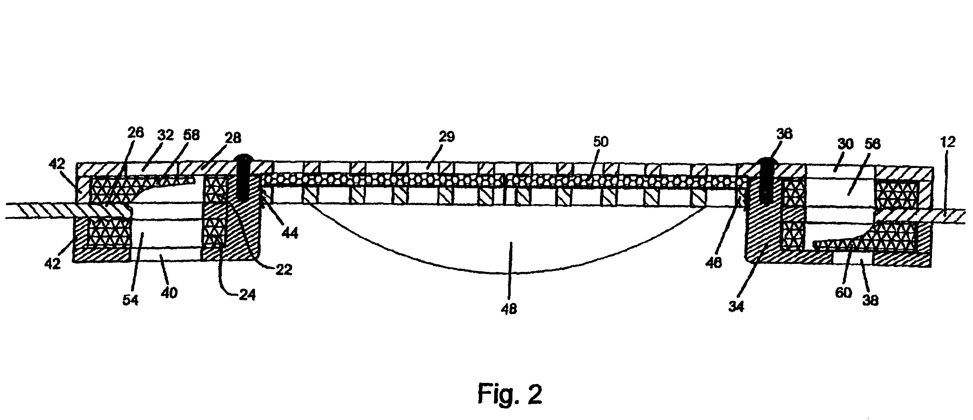

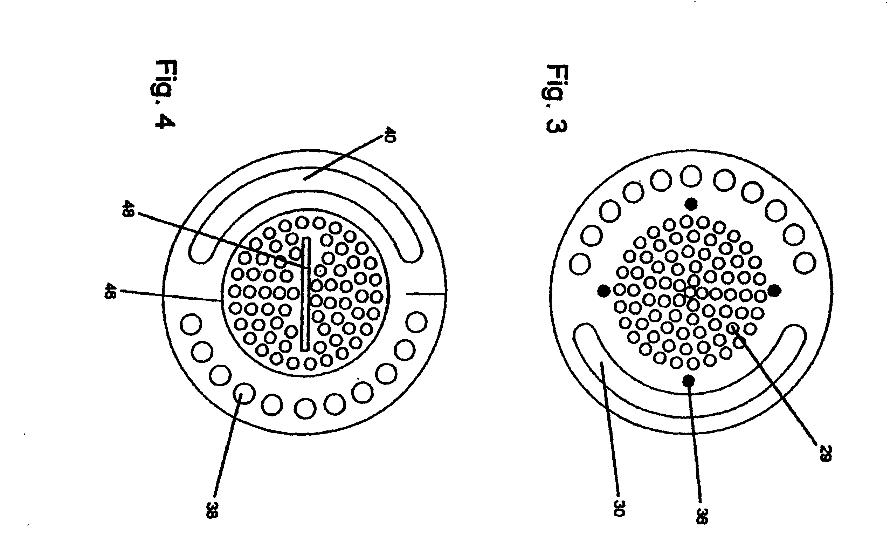

Der in Fig. 1 dargestellte Sterilisierbehälter entspricht in seinem Grundaufbau dem Sterilisierbehälter gemäß der DE 31 46 349 A1, und er besteht aus einem Behälterunterteil 10, einem Zwischendeckel 12, der über einen Dichtring 14 auf den nach innen eingezogenen Rand des Behälterunterteils aufgesetzt ist und einem geschlossenen Behälterdeckel 16, der den Behälterunterteil mit Zwischendeckel übergreift und einen Layrinthspalt 18 zum Medienaustausch während des Sterilisiervorganges im Sterilisator freiläßt. Der Zwischendeckel trägt zum Zwecke des Medienaustausches eine Filter-Ventil-Kombination 20, die aus den Fig. 2 bis 5 im einzelnen ersichtlich ist. Diese Filter-Ventil-Kombination 20 im Doppeldeckel erweist sich als zweckmäßig, weil diese durch den übergreifenden Behälterdeckel 16 geschützt ist. Für die Erfindung soll es jedoch vorbehalten bleiben, eine derartige Filter-Ventil-Kombination auch an anderen Stellen eines Sterilisierbehälters, beispielsweise in den Seitenwänden oder im Boden oder einem den Behälter abschließenden Deckel, unterzubringen.

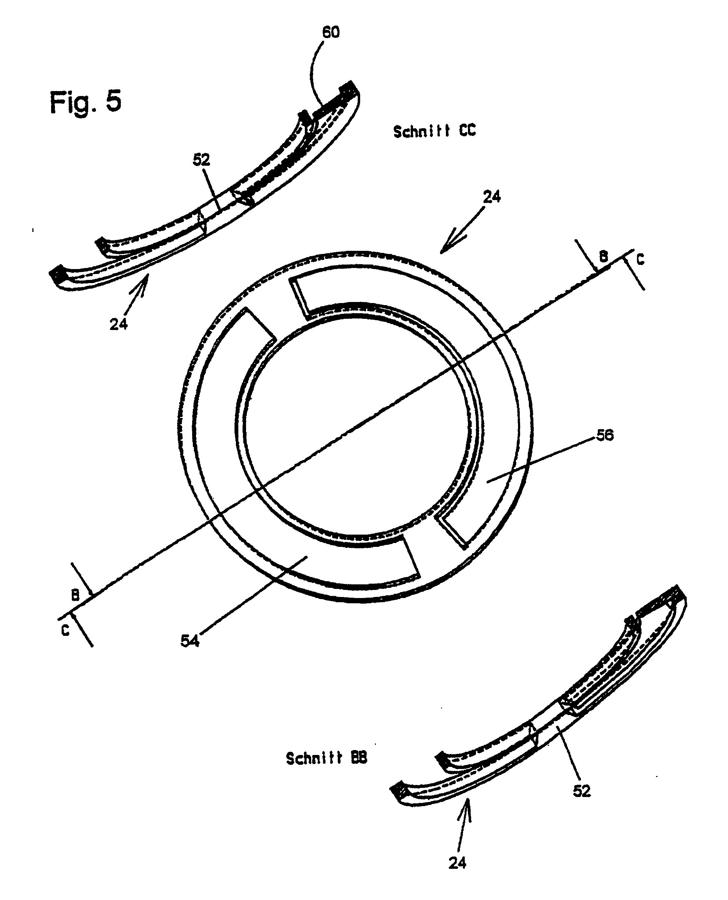

In eine kreisrunde Öffnung des Zwischendeckels 12 sind zwei Ventilringe 22, 24 eingespannt, die zwischen sich den Rand 26 der Öffnung des Zwischendeckels 12 einklemmen. Die beiden Ventilringe 22, 24 sind identisch ausgebildet, jedoch in ihrer Einbaulage um 180° verdreht und umgekehrt aufeinandergelegt (Fig. 5). Die Ventilringe könnten auch einstückig ausgebildet sein. Die Ventilringe 22, 24 tragen ein Filterhalteblech 28, welches im Mittelbereich mit Perforationslöchern 29 für den Medienaustausch versehen ist und das auf der einen Seite über dem Ventilring 22 einen Bogenschlitz 30 und auf der anderen Seite über einem Halbkreis Löcher 32 aufweist. Von unten her ist der Ventilring 22 durch einen im Querschnitt L-förmigen Ringrahmen 34 abgestützt, dessen vertikaler Schenkel mit dem Filterhalteblech 28 durch Schrauben 36 verspannt ist. Der horizontale Schenkel des Ringrahmens weist auf der einen Seite unter dem Bogenschlitz 30 Löcher 38 auf und auf der anderen Seite unter den Löchern 32 einen Bogenschlitz 40. Der Ventilring 22 besteht aus elastischem Material, und durch die Schrauben 36 erfolgt eine dichtende Verspannung gegenüber dem Rand des Zwischendeckels. Oben und unten ist der Ventilring 22 von außen her von Ringwänden 42 umschlossen, die integral mit dem Filterhalteblech 28 bzw. dem Ringrahmen 34 hergestellt sind, beispielsweise als napfartiges Ziehteil, Spritzgußteil oder Drehteil. Der vertikale Schenkel des Ringrahmens 34 trägt ein Innengewinde 44, in das eine perforierte Spannscheibe 46 mit Handgriff 48 einschraubbar ist. Zwischen dem Filterhalteblech 28 und der Spannscheibe 46 ist eine Filterscheibe 50 dichtend eingespannt.

Der Ventilring 22 weist eine Ringkammer auf, die durch Trennwände 52 in zwei kreisbogenförmige Kammern 54 bzw. 56 unterteilt ist, die sich zwischen den Trennwänden 52 über nahezu 180° erstrecken. In dem gemäß Fig. 2 linken Kreisbogenabschnitt weist der Ventilring eine von außen nach innen einstehende Lippendichtung 58 über der Kammer 54 auf, die mit einer vorbestimmten Kraft gegen das Filterhalteblech 28 vorgespannt ist und die Löcher 32 abdichtet. Im Bereich der anderen bogenförmigen Kammer 56 weist der Ventilring 22 eine von außen nach innen stehende Lippendichtung 60 auf, die gegen den horizontalen Schenkel des Ringrahmens 34 vorgespannt ist und die Löcher 38 abdichtet.

Auf diese Weise wird die Filterscheibe 50 unverrückbar gegenüber dem Zwischendeckel 12 über den Halterahmen festgelegt, der von dem Ringrahmen 34, dem Ventilring 22 und dem Filterhalteblech gebildet ist, wobei eine Verspannung über die Spannscheibe 46 erfolgt. Wenn während des Sterilisiervorganges der Differenzdruck zwischen außen und innen einen Wert überschreitet, der über den Filter 50 nicht mehr ausgeglichen werden kann, dann wird die Lippendichtung 58 vom Filterhalteblech 28 abgehoben, so daß ein Medienaustausch über die Löcher 32, die Teilkammer 54 und den Bogenschlitz 40 erfolgen kann. Wenn demgegenüber während des Sterilisiervorganges die Druckdifferenz zwischen dem Inneren des Sterilisierbehälters und dem Sterilisator einen vorbestimmten Wert überschreitet, dann wird die Lippendichtung 60 von den Löchern 38 abgehoben, und es wird ein Bypass über den Kammerabschnitt 56 und den Bogenschlitz 30 freigegeben. Während die Ventilanordnung öffnet und durch die elastische Vorspannung der Lippendichtungen selbsttätig wieder schließt, bleibt das Filterhalteblech unverrückbar fest eingespannt, so daß keine Störungen auftreten können, wenn Teile des Behälterinhalts in die Nähe des Filters gelangen. Um auch im Bereich der Lippendichtungen 58 und 60 Störungen zu vermeiden und eine einwandfreie Ventilfunktion auch dann zu gewährleisten, wenn innen oder außen Gegenstände in die Nähe der Durchtrittsöffnungen oder Schlitze gelangen, können diese zum Schutz überbrückt sein. Dies kann beispielsweise dadurch geschehen, daß beim Ausstanzen der Löcher oder Schlitze Brückenstege über den Löchern oder Schlitzen ausgeformt werden.

Gemäß dem Ausführungsbeispiel nach Fig. 6 bis 9 ist der Zwischendeckel 12A als oberes Filterhalteblech ausgebildet und besitzt zu diesem Zweck eine Perforation 29A, unter der die Filterscheibe 50A verspannt ist. Aus den Fig. 6 und 9 ist ersichtlich, daß es sich hierbei um beliebige Filter handeln kann, nämlich, wie auf der rechten Seite skizziert, um einen dünnen Flachfilter, und, wie aus der linken Hälfte ersichtlich, um einen dickeren Filter, beispielsweise einen Keramikfilter oder Volumenfilter.

Dieser Filter 50A ist von unten her durch ein Filterhalteblech gegen den Zwischendeckel 12A verspannt, wobei ein Dichtring 64 eine Abdichtung zwischen Filter 50A und Zwischendeckel 12A am äußeren Rand bewirkt. Dieses Filterhalteblech ist an dem Zwischendeckel 12A am äußeren Rand auf beliebige Weise fixiert, beispielsweise durch Einklemmen oder Einschrauben. Wichtig dabei ist, daß bei absichtlicher Zerlegung (z.B. Filterwechsel oder -inspektion) keinerlei Hohlräume verbleiben. Das sichert ausgezeichnete Reinigungsmöglichkeiten ohne Waschschatten. Das Filterhaltelblech 62 ist mit Perforationslöchern 66 ausgestattet. Rings um die Filterscheibe 50A ist zwischen dem Zwischendeckel 12A und dem Filterhalteblech 62 eine Ringkammer 68 ausgebildet, in der ein Ventilring 70 zwischen Zwischendeckel 12A und Filterhalteblech 62 verspannt ist. Zwei diametral gegenüberliegende Trennwände 72 des Ventilrings 70 unterteilen die Ringkammer 68 in zwei Kreisbogenabschnitte. In der gemäß Fig. 6 bis 8 links liegenden Bogenkammer trägt der Ventilring 70 Dichtlippen 74, die gegen den Zwischendeckel 12A vorgespannt sind und die in diesem Bereich angeordnete Öffnung 76 des Zwischendeckels abschließen. Auf der gemäß Fig. 6 bis 8 rechten Seite weist der Ventilring 70 nach unten gegen das Filterhalteblech 62 vorgespannte Dichtlippen 78 auf, die nach unten gegen das Filterhalteblech 62 vorgespannt sind und die Öffnungen 80 in diesem Bogenabschnitt abdecken. Unter den Öffnungen 76 weist das Filterhalteblech 62 Öffnungen 82 auf, über die bei Auftreten eines äußeren Überdrucks der Medienaustausch erfolgen kann, nachdem die Dichtlippen 74 von den Öffnungen 76 abgehoben sind. Über den Öffnungen 80 weist der Zwischendeckel 12A Öffnungen 84 auf, über die der Medienaustausch erfolgen kann, wenn durch inneren Überdruck die Dichtlippe 78 von den Öffnungen 80 abgehoben ist.

Die Fig. 10 zeigt die Querschnittsansicht eines Sterilisierbehälters, bei dem im Zwischendeckel 12A eine Filter-Einlaßventil-Kombination angeordnet ist. Diese entspricht im wesentlichen dem Aufbau nach Fig. 6 bis 9 mit dem Unterschied, daß eine durchgehende, ungeteilte Ringkammer 68B vorgesehen ist und der Ventilring 70B durchgehend in der Weise ausgebildet ist, wie dies in Fig. 6 für die linke Ringkammer dargestellt ist. Das heißt, seine Dichtlippen 74B liegen über den gesamten Kreisbogenbereich dem Zwischendeckel 12A an und verschließen die Öffnungen 76B des Zwischendeckels 12A. Beim Auftreten eines äußeren Überdrucks hebt die Dichtlippe 74B vom Zwischendeckel ab und gibt den Weg zwischen den Öffnungen 76B und den Öffnungen 82B frei.

Bei diesem Ausführungsbeispiel nach Fig. 10 ist der Boden 86 des Behälterunterteils 10 mit einer nach oben konvexen Auswölbung versehen, und der Bodenrand ist nach unten abgeschrägt, so daß sich ein vertieft liegender Ringkanal ergibt, der mit Auslaßöffnungen 88 versehen ist. Gegen diese Auslaßöffnungen 88 liegt von unten her die Dichtlippe 90 eines Ventilrings 92 an, die beim Auftreten einer vorbestimmten Druckdifferenz abhebt und damit gleichzeitig Luft/Dampf ausströmen sowie Kondenswasser austreten läßt, das sich in dem Ringkanal angesammelt hat. Der Ventilring 92 ist in einem Standrahmen 94 eingespannt. Diese Anordnung ergibt insbesondere mit dem für die Erfindung vorgesehenen dichten Filter Vorteile, weil dann zuerst das über dem Ventil stehende Wasser herausgedrückt wird. Die folgende starke Strömung nach unten/außen reißt dann vereinzelte, noch hängende Tropfen mit und ermöglicht so die einwandfreie Trocknung bei der Dampfsterilisation.

Bei dem Ausführungsbeispiel nach Fig. 11 bis 13 ist ein Ventilring 22A mit seiner äußeren Ringnut 24 auf den Rand 26 der Öffnung des Zwischendeckels 12 eingeschoben. Dieser Ventilring 22A ist zwischen einem oberen Filterhalteblech 28A und einem unteren Ringrahmen 34A, der dem Ringrahmen 34 gemäß Fig. 2 entspricht, eingespannt. Das Filterhalteblech 28A ist über Schrauben 36 mit dem vertikalen Schenkel des Ringrahmens 34 verschraubt. Der vertikale Schenkel des Ringrahmens 34A weist im Mittelabschnitt eine im Querschnitt dreieckige Rippe 96 auf. Diese Rippe 96 wirkt als Ventilsitz für eine Dichtlippe 98, welche nach innen von dem Ventilring 22A über den gesamten Umfang verlaufend vorsteht. Die Dichtlippe 98 ist als durchgehende Ringlippe ausgebildet und befindet sich in einer Ringkammer 100 zwischen Ventilring 22A und dem vertikalen Schenkel des Ringrahmens 34A. Das Filterhalteblech weist über der Ringkammer 100 Öffnungen 102 auf, und der horizontale Schenkel des Rahmens 34A weist Öffnungen 104 auf. Wie bei dem Ausführungsbeispiel nach Fig. 2 ist in den Rahmen 34A eine perforierte Spannscheibe 46 eingeschraubt, die die Filterscheibe 50 gegen das Filterhalteblech verspannt. In der in Fig. 11 dargestellten Stellung liegt die Dichtringlippe 98 dem Ventilsitz, d.h. der Rippe 96, von oben her dichtend an. Beim Auftreten einer vorbestimmten Druckdifferenz mit einem gegenüber dem Außendruck erhöhten Innendruck wird die Dichtlippe 98 abgehoben und gibt den Strömungs-Bypass-Weg von den Öffnungen 104 nach den Öffnung 102 frei. Wenn sich eine vorbestimmte Druckdifferenz in umgekehrter Richtung aufbaut, dann rutscht die Lippe 98 an der Rippe vorbei und öffnet den Strömungsweg von den Öffnungen 102 nach den Öffnungen 104. Wenn dann dieser Überdruck abgebaut ist, liegt die Dichtlippe 98 aufgrund ihrer Elastizität der Rippe 96 von unten her an und bewirkt einen keimdichten Abschluß.

Auch bei diesem Ausführungsbeispiel gibt es außer der Dichtlippe innerhalb der allseitig abgeschlossenen Ringkammer 100 keine bewegten Teile, denn die Filter-Ventil-Kombination ist unverrückbar mit dem Zwischendeckel 12 verspannt. Natürlich könnte diese Filter-Ventil-Kombination auch in einer Behälterseitenwand oder einem auf das Behälterunterteil dichtend aufgesetzten Deckel angeordnet sein.

Ein weiteres Ausführungsbeispiel ist in den Fig. 14 bis 16 dargestellt. Hier ist das obere Filterhalteblech 28B am Umfang auf einem Dichtungsring 106 abgestützt, der aus Weichgummi oder geschäumtem porösem Material besteht und auf dem Öffnungsrand des Zwischendeckels 12 aufliegt. Das Filterhalteblech ist, wie bei dem Ausführungsbeispiel nach Fig. 2 und 11, mit einem Ringrahmen 34B verschraubt, in den wiederum eine perforierte Spannscheibe 46 eingeschraubt ist. Der horizontale Schenkel des Ringrahmens 34B trägt einen gegen den Rand des Zwischendeckels 12 von unten her verspannten Dichtungsring 108 mit einer nach innen weisenden Dichtungslippe 110, die im Ruhezustand Öffnungen 112 der Spannscheibe 46 abdeckt.

Wenn sich während des Sterilisiervorganges der Außendruck gegenüber dem Innendruck aufbaut, dann wird bei Erreichen einer vorbestimmten Druckdifferenz der Dichtungsring 28B zusammengequetscht, und mit dem Filterhalteblech wird der Ringrahmen 34 nach unten gedrückt, wodurch der Dichtungsring 108 von dem Öffnungsrand des Zwischendeckels 12 abgehoben wird, so daß über die Öffnungen 102 im Filterhalteblech und die ringförmige Öffnung über den Dichtungsring 108 ein Bypass geschaffen wird. Beim Auftreten einer Druckdifferenz in der umgekehrten Richtung wird die Dichtlippe 110 von den Öffnungen 112 abgehoben, und es wird über die Öffnungen 102 ein Bypass in umgekehrter Richtung geschaffen. Hierbei erfolgt zwar eine Bewegung des gesamten Filteraufbaus mit Halterahmen beim Auftreten eines äußeren Überdrucks, jedoch kann durch geeignete Abdeckungen verhindert werden, daß der Behälterinhalt die Funktion der Ventilanordnung stört oder blockiert.

Die Fig. 17 bis 20 zeigen ein weiteres Ausführungsbeispiel. Hierbei ist ein Dichtungsring 114 aus Weichgummi mit einer äußeren Ringnut auf den Rand einer Deckelöffnung des Zwischendeckels 12 aufgeschoben. Auf dem Dichtungsring 114 liegt der Rand des Filterhaltebleches 28. Von unten her stützt sich gegen den Dichtungsring 114 der horizontale Schenkel des Ringrahmens 34 ab, in den die Spannscheibe 46 eingeschraubt ist, die die Filterscheibe 50 gegen das Filterhalteblech drückt. Zwischen dem Dichtungsring 114 und dem Ringrahmen 34 sind zwei kreisbogenförmige Kammern 54 bzw. 56 ausgebildet, die durch nach innen stehende Trenn-wände 116 des Dichtungsringes 114 gegeneinander abgedichtet sind. Die Kammer 56 steht über Öffnungen 118 der Filterhaltescheibe 28 in Verbindung mit außen, und die Kammer 54 steht über Öffnungen 120 der Spannscheibe 46 in Verbindung mit dem Behälterinneren.

Beim Auftreten eines äußeren Überdrucks wird der obere Teil des Dichtringes 114 zusammengequetscht, und es wird der horizontale Schenkel des Ringrahmens 34 von diesem Dichtring abgehoben, wodurch ein Bypass über die Öffnungen 118 geschaffen wird. Umgekehrt wird beim Auftreten eines Überdrucks von innen her die Unterseite des Dichtungsringes 114 zusammengequetscht, wodurch das Filterhalteblech mit seinem Rand vom Dichtungsring abgehoben wird und einen Bypass über die Öffnungen 120 freigibt.

Der erfindungsgemäße Sterilisierbehälter ist nicht nur für eine Dampfsterilisation geeignet, sondern es ist auch möglich, durch Gas oder Plasma eine Sterilisation vorzunehmen. Durch den erfindungsgemäßen Sterilisierbehälter wird die Sterilität bis zum absichtlichen Öffnen aufrechterhalten, weil durch Anwendung der Erfindung- ohne die Sterilisation zu behindern - wesentlich bessere, dichtere Filter zum Einsatz kommen können.

Die Erfindung ist nicht nur für klinisches Sterilgut anwendbar, sondern auch für andere beliebige, zu sterilisierende Güter, beispielsweise in der pharmazeutischen Industrie.

Um die Durchtrittsöffnungen zu schützen und um zu verhindern, daß Gegenstände in die Öffnungen eindringen und die Funktion der Ventile beeinträchtigen, kann eine Schutzabdeckung vorgesehen werden, wobei diese Schutzabdeckung durch Überbrückungen der Löcher beim Ausstanzen erfolgen kann.

Die bevorzugte Ausführung ist die kreisförmige Ausführung, jedoch sind auch rechteckige Filter-Ventil-Kombinationen möglich.

### Bezugszeichenliste

- 10: Behälterunterteil
- 12 (A): Zwischendeckel
- 14: Dichtring
- 16: Behälterdeckel
- 18: Labyrinthspalt
- 20: Filter-Ventil-Kombination
- 22: Ventilring
- 24: Ventilring
- 26: Rand
- 28: Filterhalteblech
- 29: Perforation
- 30: Bogenschlitz
- 32: Löcher
- 34: Ringrahmen
- 36: Schrauben
- 38: Löcher
- 40: Bogenschlitz
- 42: Ringwände
- 44: Innengewinde
- 46: Spannscheibe
- 48: Handgriff
- 50: Filterscheibe
- 52: Trennwände
- 54,56: kreisbogenförmige Kammern
- 58,60: Lippendichtung
- 62: Filterhalteblech
- 64: Dichtring
- 66: Perforationslöcher
- 68: Ringkammer
- 70: Ventilring
- 72: Trennwände
- 74: Dichtlippe
- 76: Öffnungen
- 78: Dichtlippe
- 80: Öffnungen
- 82: Öffnungen
- 84: Öffnungen
- 86: Boden
- 88: Auslaßöffnungen
- 90: Dichtlippe
- 92: Ventilring
- 94: Standrahmen
- 96: Rippe
- 98: Dichtlippe
- 100: Ringkammer
- 102: Öffnungen
- 104: Öffnungen
- 106: Dichtring
- 108: Dichtring
- 110: Dichtlippe
- 112: Öffnungen
- 114: Dichtring
- 116: Wände
- 118: Öffnungen
- 120: Öffnungen

## Patentansprüche

1. Sterilisierbehälter zur Aufnahme von OP-Instrumenten oder anderweitigem Sterilisiergut während der Sterilisation und zur Sterilitätserhaltung während der Lagerung und beim Transport mit den folgenden Merkmalen:
- der Behälter weist wenigstens eine Öffnung zur Ermöglichung des Medienaustausches während des Sterilisiervorganges auf;
- in die Öffnung ist ein Filter (50) dichtend eingesetzt, der wenigstens einen begrenzten Medienaustausch zuläßt und während der Lagerung eine Barriere gegen Mikroorganismen bildet;
- ein selbstschließendes Einlaßventil (58) am Behälter, welches beim Überschreiten einer vorbestimmten Druckdifferenz zwischen Außen- und Innendruck nach dem Behälter-inneren öffnet.
- ein selbstschließendes Auslaßventil (60) am Behälter, welches beim Überschreiten einer vorbestimmten Druckdifferenz zwischen Innen- und Außendruck nach außen öffnet;
- der Filter ist in einem Halterahmen (34) eingespannt und allseitig formschlüssig gegenüber diesem abgestützt;
- der Halterahmen ist am Rand der Behälteröffnung eingespannt;
- wenigstens das Einlaßventil (58) ist innerhalb des Halterahmens angeordnet.

2. Sterilisierbehälter nach Anspruch 1,
**dadurch gekennzeichnet, daß** der Filter und beide Ventile in einer einzigen, gemeinsamen Öffnung des Behälters angeordnet und beide Ventile in den Halterahmen integriert sind.

3. Sterilisierbehälter nach den Ansprüchen 1 und 2,
**dadurch gekennzeichnet, daß** die Filter-Ventil-Kombination (20) mit ihrem Halterahmen (34) am Rand (26) der Öffnung verspannt und allseitig formschlüssig abgestützt ist.

4. Sterilisierbehälter nach Anspruch 1,
**dadurch gekennzeichnet, daß** der Filter (50) beidseitig durch je ein perforiertes Blech abgestützt ist.

5. Sterilisierbehälter nach Anspruch 4,
**dadurch gekennzeichnet, daß** die Filter-Ventil-Kombination (20) kreisförmig ausgebildet ist, daß ein eine Filterscheibe (50) oben abstützendes Filterhalteblech (28) mit dem ringförmigen Halterahmen (34) verspannt ist und daß eine den Filter (50) von der anderen Seite abstützende Spannscheibe in ein Innengewinde (44) des ringförmigen Halterahmens (34) dichtend eingesetzt, z.B. eingeschraubt oder eingedrückt oder mit einem federnden Verschluß angedrückt ist.

6. Sterilisierbehälter nach Anspruch 5,
**dadurch gekennzeichnet, daß** die Ventile als Lippenventile (58, 60) kreisbogenförmig ausgebildet und innerhalb des ringförmigen Halterahmens (34) ausgebildet sind.

7. Sterilisierbehälter nach Anspruch 6,
**dadurch gekennzeichnet, daß** die Ventile von einem zweiteiligen, umlaufenden Dichtring (22, 24) gebildet sind, der auf dem Öffnungsrand (26) aufgeklemmt ist.

8. Sterilisierbehälter nach Anspruch 7,
**dadurch gekennzeichnet, daß** die Ventilringe (22, 24) zusammen zwei kreisbogenförmige Kammern (54, 56) bilden, die durch Trennwände (52) voneinander getrennt sind und daß die eine kreisbogenförmige Kammer (54) oben mit einer Lippendichtung (58) ausgestattet ist, die gegen Öffnungen (32) des Filterhalteblechs (28) wirkt, während in der anderen kreisbogenförmigen Kammer (56) eine Lippendichtung (60) gegen Rahmenöffnungen (38) vorgespannt ist, die nach dem Behälterinneren führen.

9. Sterilisierbehälter nach Anspruch 1,
**dadurch gekennzeichnet, daß** der Halterahmen von einem Zwischendeckel (12A) oder einem anderen Behälterteil und einem unter Zwischenfügung eines Filters (50A) dagegen verspannten Filterhalteblech (72) gebildet ist und daß die Lippendichtungen (74, 78) in der zwischen Zwischendeckel (12A) und Filterhalteblech (62) gebildeteten Ringkammer (68) angeordnet sind, die umfangsmäßig durch Trennwände (72) unterteilt ist, wobei in einer Teilringkammer die Dichtlippen (74) gegen Öffnungen (76) des Zwischendeckels (12A) anliegen und in der anderen Teilringkammer gegen Öffnungen (80) des Filterhalteblechs.

10. Sterilisierbehälter nach Anspruch 1,
**dadurch gekennzeichnet, daß** ein rings umlaufendes Einlaßventil (74) in einer den Filter (50) umschließenden Kammer im Deckel oder der Behälterwand angeordnet ist und daß das Auslaßventil (90) als Kondensat-Ventil in einer ringförmigen Bodenvertiefung des Behälterbodens angeordnet ist.

11. Sterilisierbehälter nach Anspruch 10,
**dadurch gekennzeichnet, daß** das Auslaßventil von einem am Behälterboden verspannten Ventilring (92) gebildet ist, der eine nach innen stehende Ventillippe (90) aufweist, welche gegen Bodenöffnungen (88) elastisch vorgespannt ist und daß diese Anordnung gleichzeitig als Kondensat-Ableitventil arbeitet.

12. Sterilisierbehälter nach einem der Ansprüche 1 bis 9,
**dadurch gekennzeichnet, daß** im Ringrahmen (34A) ein auf dem Behälterrand (26) verspannter Ventilring (22A) eingesetzt ist, der eine nach innen gerichtete Dichtlippe (98) trägt, die mit einer Ringrippe (96) des Ringrahmens (34A) zusammenwirkt.

13. Sterilisierbehälter nach Anspruch 12,
**dadurch gekennzeichnet, daß** die Dichtlippe (98) beim Überschreiten eines vorbestimmten Differenzdrucks über die Rippe (96) unter Öffnung eines Bypass-Weges aushebbar ist.

14. Sterilisierbehälter nach Anspruch 1,
**dadurch gekennzeichnet, daß** der Ringrahmen (34B) gegenüber dem Behälterrand unter Öffnung eines Bypass-Weges aushebbar ist.

15. Sterilisierbehälter nach Anspruch 14,
**dadurch gekennzeichnet, daß** der Rahmen einen aus Weichgummi bestehenden Dichtungsring (114) aufweist, der beim Auftreten eines vorbestimmten Differenzdruckes unter Freigabe eines Bypass-Weges zusammenquetschbar ist.

16. Sterilisierbehälter nach den Ansprüchen 14 und 15,
**dadurch gekennzeichnet, daß** die Weichgummidichtung (114) eine äußere Ringnut aufweist, mit der sie in den Öffnungsrand des Behälters eingesetzt ist und daß die zwischen Dichtungsring (114) und Ringrahmen (34) gebildete Ringkammer durch Wände (116) in zwei kreisbogenförmige Kammern unterteilt ist, die einerseits über Öffnungen (118) des Filterhalteblechs (28) und andererseits über Öffnungen (120) einer Spannscheibe nach außen bzw. dem Behälterinneren führen.

17. Sterilisierbehälter nach Anspruch 14 und 15,
**dadurch gekennzeichnet, daß** der als Weichgummiring ausgebildete Ventilring zwischen Öffnungsrand und Filterhalteblechrand angeordnet ist und daß eine umlaufende, mit umlaufender Dichtlippe (110) versehene Ringdichtung (108) zwischen Behälterrand und Rahmen angeordnet ist und nach dem Behälterinneren führende Öffnungen (112) abschließt.

18. Sterilisierbehälter nach Anspruch 17,
**dadurch gekennzeichnet, daß** der Ringrahmen (34B) mit dem Dichtungsring (108) nach innen unter Zusammenquetschen des Dichtungsringes (28B) aushebbar ist.

19. Sterilisierbehälter nach einem der Ansprüche 1 bis 18,
**dadurch gekennzeichnet, daß** der Filter als Volumenfilter ausgebildet ist.

20. Sterilisierbehälter nach den Ansprüchen 1 und 19,
**dadurch gekennzeichnet daß** der Volumenfilter in einem in sich gekapselten Gehäuse untergebracht ist, das in den Rahmen (34) dichtend eingesetzt, z.B. eingeschraubt ist.

## Claims

1. Sterilisation vessel to hold operating theatre instruments or other material for sterilisation during sterilisation, and to maintain sterilisation during storage and transport, having the following characteristics:
- the vessel has one or more openings to facilitate the interchange of media during the sterilisation process;
- inserted in the opening with sealing is a filter (50) which allows at lead limited interchange of media and forms a barrier against micro-organisms during storage;
- a self-closing inlet valve (58) on the vessel, opening towards the inside of the vessel if a preset pressure difference between external and internal pressure is exceeded;
- a self-closing outlet valve (60) on the vessel, opening outwards if a preset pressure difference between internal and external pressure is exceeded;
- the filter is clamped in a mounting frame (34) against which it is positively supported on all sides;
- the mounting frame is clamped to the edge of the vessel opening;
- at least the inlet valve (58) is located within the mounting frame.

2. Sterilisation vessel according to claim 1, **characterised in that** the filter and both valves are arranged in a single, common opening of the vessel, and both valves are integrated in the mounting frame.

3. Sterilisation vessel according to claims 1 and 2, **characterised in that** the filter-valve combination (20) is braced at the edge (26) of the opening and positively supported on all sides by its mounting frame (34).

4. Sterilisation vessel according to claim 1, **characterised in that** the filter (50) is supported on each side by a perforated plate.

5. Sterilisation vessel according to claim 4, **characterised in that** the filter-valve combination (20) is circular in form, that a filter retaining plate (28) supporting a filter disc (50) at the top is braced by the annular mounting frame (34), and that a clamping disc supporting the filter (50) from the other side is inserted with sealing in an internal thread (44) of the annular mounting frame (34), e.g. screwed or pressed in or pressed on by a sprung closure.

6. Sterilisation vessel according to claim 5, **characterised in that** the valves are in the form of circular lip valves (58, 60) and are provided within the annular mounting frame (34).

7. Sterilisation vessel according to claim 6, **characterised in that** that valves are formed by a two-part, all-round seal ring (22, 24), which is clamped on to the opening edge (26).

8. Sterilisation vessel according to claim 7, **characterised in that** the vulve rings (22, 24) together form two circular chambers (54, 56), separated from one another by partition walls (52), and that one circular chamber (54) is provided at the top with a lip seal (58) which acts against openings (32) of the filter retaining plate (28), while in the other circular chamber (56) a lip seal (60) is pre-stressed against frame openings (38) which lead to the inside of the vessel.

9. Sterilisation vessel according to claim 1, **characterised in that** the mounting frame is formed by an intermediate cover (12A) or another part of the vessel and a filter retaining plate (72) [sic] braced against it with an interposed filter (50A), and that the lip seals (74, 78) are located in the annular chamber (68) formed between the intermediate cover (12A) and the filter retaining plate (62), which is divided circumferentially by partition walls (72), while in one annular chamber section the seal lips (74) fit up against openings (76) of the intermediate cover (12A) and in the other annular chamber section they fit up against openings (80) of the filter retaining plate.

10. Sterilisation vessel according to claim 1, **characterised in that** an inlet valve (74) running all round is mounted in a chamber, which encloses the filter (50), in the cover or the vessel wall, and that the outlet valve (90) is fitted as a condensate valve in an annular base recess of the vessel base.

11. Sterilisation vessel according to claim 10, **characterised in that** the outlet valve is formed by a valve ring (92) braced against the vessel base and with a valve lip (90) facing inwards, which is elastically pre-stressed against base openings (88), and that this assembly operates simultaneously as condensate drain valve.

12. Sterilisation vessel according to any of claims 1 to 9, **characterised in that**, inserted in the annular frame (34A) is a valve ring (22A) braced against the vessd edge (26) and carrying an inwards-directed seal lip (98) which acts in conjunction with an annular rib (96) of the annular frame (34A).

13. Sterilisation vessel according to claim 12, **characterised in that** the seal lip (98), if a preset pressure difference is exceeded, can be lifted over the rib (96) to open up a bypass.

14. Sterilisation vessel according to claim 1, **characterised in that** the annular frame (34B) can be lifted relative to the vessel edge to open up a bypass.

15. Sterilisation vessel according to claim 14, **characterised in that** the frame has a seal ring (114) made of flexible rubber which can be squeezed together to release a bypass if a preset pressure difference occurs.

16. Sterilisation vessel according to claims 14 and 15, **characterised in that** the flexible rubber seal (114) has an external annular groove by which it is inserted into the opening edge of the vessel, and that the annular chamber formed between the seal ring (114) and the annular frame (34) is divided by walls (116) into two circular chambers which lead on one side via openings (118) in the filter retaining plate (28) and on the other side via openings (120) in a clamping disc to the outside or inside of the vessel respectively.

17. Sterilisation vessel according to claims 14 and 15, **characterised in that** the valve ring in the form of a flexible rubber ring is located between the opening edge and the filter retaining plate edge, and that an all-round ring seal (108) with an all-round seal lip (110) is fitted between the vessel edge and the frame, and closes openings (112) leading to the inside of the vessel.

18. Sterilisation vessel according to claim 17, **characterised in that** the annular frame (34B) with the seal ring (108) may be lifted inwards with the seal ring (28B) being squeezed together.

19. Sterilisation vessel according to any of claims 1 to 18, **characterised in that** the filter is in the form of a volume filter.

20. Sterilisation vessel according to claims 1 and 19, **characterised in that** the volume filter is accommodated in a self-encapsulated housing which is inserted with sealing. e.g. by screwing, into the frame (34).

## Revendications

1. Récipient stérilisateur pour accueillir des instruments chirurgicaux ou autre produit à stériliser au cours de la stérilisation et pour conserver le caractère stérile pendant le stockage et le transport, présentant les caractéristiques suivantes :
- le stérilisateur comporte au moins une ouverture permettant le remplacement du milieu au cours du processus de stérilisation ;
- un filtre (50) est enchâssé dans l'ouverture de façon étanchéifiante, qui autorise au moins un remplacement limité du milieu et qui forme pendant le stockage une barrière contre les microorganismes
- une soupape d'admission à fermeture automatique (58) est prévue au niveau du récipient, qui s'ouvre sur l'intérieur du récipient en cas de dépassement d'une différence de pression prédéterminée entre pression extérieure et pression intérieure,
- une soupape d'évacuation à fermeture automatique (60) est prévue au niveau du récipient, qui s'ouvre sur l'extérieur en cas de dépassement d'une différence de pression prédéterminée entre pression intérieure et pression extérieure,
- le filtre est encastré dans un cadre de support (34) et bute de tous côtés contre celui-ci par engagement de forme ;
- le cadre de support est encastré au niveau du bord de l'ouverture du récipient ;
- au moins la soupape d'admission (58) est disposée à l'intérieur du cadre de support.

2. Récipient stérilisateur selon la revendication 1, **caractérisé en ce que** le filtre et les deux soupapes sont disposés dans une seule ouverture commune du récipient et **en ce que** les deux soupapes sont intégrées au cadre de support.

3. Récipient stérilisateur selon les revendications 1 et 2, **caractérisé en ce que** la combinaison filtre-soupape(20) est bloquée avec son cadre de support (34) au niveau du bord (26) de l'ouverture et **en ce qu'**elle est butée de tous côtés par engagement de forme.

4. Récipient stérilisateur selon la revendication 1, **caractérisé en ce que** le filtre (50) est buté des deux côtés par une tôle perforée respective.

5. Récipient stérilisateur selon la revendication 4, **caractérisé en ce que** la combinaison filtre-soupape (20) est concue de façon circulaire, **en ce qu'**une plaque métallique de support de filtre (28) butant par le haut un disque filtrant (50) est bloquée avec le cadre de support en forme d'anneau (34), et **en ce qu'**un disque de serrage butant le filte (50) de l'autre côté est inséré dans un taraudage intérieur (44) du cadre de support en forme d'annean (34) de façon étanchéifiante, par ex. vissé ou enfoncé, ou encore comprimé avec un dispositif de fermeture à ressort.

6. Récipient stérilisateur selon la revendication 5, **caractérisé en ce que** les soupapes sont conçues en arcs de cercles sous forme de soupapes à bec (58, 60) et **en ce qu'**elles sont formées à l'intérieur du cadre de support en forme d'anneau (34).

7. Récipient stérilisateur selon la revendication 6, **caractérisé en ce que** les soupapes sont formées par une bague d'étanchéité périphérique en deux parties (22, 24), qui est coincée sur le bord d'ouverture (26).

8. Récipient stérilisateur selon la revendication 7, **caractérise en ce que** les bagues des soupapes (22, 24) forment ensemble deux chambres en arcs de cercles (54, 56), qui sont séparées l'une de l'autre par des cloisons (52), et **en ce que** l'une des chambres en arc de cercle (54) est pourvue en haut d'un joint à lèvre (58) qui fonctionne par rapport aux ouvertures (32) de la plaque métallique de support du filtre (28), tandis qu'un joint à lèvre (60) est pré-tendu dans l'autre chambre en arc de cercle (56), qui fonctionne par rapport aux ouvertures du cadre (38) qui mènent vers l'intérieur du récipient.

9. Récipient stérilisateur selon la revendication 1, **caractérisé en ce que** le cadre de support est formé par un couvercle intermédiaire (12A) ou par une autre partie du récipient, et une plaque métallique de support de filtre (72) avec intercalage d'un filtre (50A) qui la bloque, et **en ce que** les joints à lèvre (74, 78) sont disposés dans la chambre annulaire (68) formée entre le couvercle intermédiaire (12A) et la plaque métallique de support du filtre (62), qui, au niveau de sa périphérie, est compartimentée par des cloisons (72), les lèvres d'étanchéité (74) reposant ce faisant contre les ouvertures (76) du couvercle intermédiaire (12A) dans une chambre annulaire partielle et contre les ouvertures (80) de la plaque métallique de support du filtre dans l'autre chambre annulaire partielle.

10. Récipient stérilisateur selon la revendication 1, **caractérisé en ce qu'**une soupape d'admission annulaire périphérique (74) dans une chambre renfermant le filtre (50) est disposée dans le couvercle ou dans la paroi du récipient, et **en ce que** la soupape d'éacuation (90) sous forme d'une soupape à condensat est disposée dans un creux de fond en forme d'anneau du fond du récipient.

11. Récipient stérilisateur selon la revendication 10, **caractérisé en ce que** la soupape d'évacuation est formée d'une bague de soupape bloquée au niveau du fond du récipient (92), qui comporte un bec de soupape dirigé vers l'intérieur (90), qui est pré-tendu élastiquement contre les ouvertures du fond (88), et **en ce que** cette installation fonctionne simultanément comme soupape de purge du condensat.

12. Récipient stérilisateur selon l'une des revendications 1 à 9, **caractérisé en ce qu'**une bague de soupape (22A) bloquée sur le bord du récipient (26) est enchâssée dans le cadre en forme d'anneau (34A), qui porte une lèvre d'étanchéité orientée vers l'intérieur (98), qui coopère avec une nervure annulaire (96) du cadre en forme d'anneau (34A).

13. Récipient stérilisateur selon la revendication 12, **caractérisé en ce que** la lèvre d'étanchéité (98), en cas de dépassement d'une différence de pression prédéterminée, peut être soulevée au-dessus de la nervure (96) en ouvrant un circuit de dérivation.

14. Récipient stérilisateur selon la revendication 1, **caractérisé en ce que** le cadre en forme d'anneau (34B) peut être soulevé par rapport au bord du récipient en ouvrant un circuit de dérivation.

15. Récipient stérilisateur selon la revendication 14, **caractérisé en ce que** le cadre comporte une bague d'étanchéité constituée de caoutchouc mou (114) capable de s'écraser lorsque survient une différence de pression prédéterminée, en libérant un circuit de dérivation.

16. Récipient stérilisateur selon les revendications 14 et 15, **caractérisé en ce que** le joint de caoutchouc mou (114) comporte une rainure annulaire extérieure, avec laquelle il est posé dans le bord d'ouverture du récipient, et **en ce que** la chambre annulaire formée entre la bague d'étanchéité (114) et le cadre en forme d'anneau (34) est compartimentée par des parois (116) en deux chambres formant des arcs de cercles, qui mènent vers l'extérieur ou vers l'intérieur du récipient, d'un côté via les ouvertures (118) de la plaque métallique de support du filtre (28) et de l'autre côté via les ouvertures (120) d'un disque de serrage.

17. Récipient stérilisateur selon les revendications 14 et 15, **caractérisé en ce que** la bague de soupape conçue sous forme d'une bague de caoutchouc mou est disposée entre le bord d'ouverture et le bord de la plaque métallique de support du filtre, et **en ce qu'**un joint d'étanchéité annulaire (108) doté d'une lèvre d'étanchéité périphérique (110) est disposé entre le bord du récipient et le cadre, et **en ce qu'**il ferme les ouvertures menant vers l'intérieur du récipient (112).

18. Récipient stérilisateur selon la revendication 17, **caractérisé en ce que** le cadre en forme d'anneau (34B) peut être soulevé avec la bague d'étanchéité (108) vers l'intérieur en écrasant la bague d'étanchéité (28B).

19. Récipient stérilisateur selon l'une des revendications 1 à 18, caractérisé ce que le filtre est conçu comme filtre volumétrique.

20. Récipient stérilisateur selon les revendications 1 et 19, **caractérisé en ce que** le filtre volumétrique est logé dans un boîtier totalement étanche, qui est inséré ou vissé de façon étanchéifiante dans le cadre (34).
